# EUROPEAN PATENT SPECIFICATION

(11) **EP 1 137 445 B1**
(45) Date of publication and mention of the grant of the patent: **09.04.2003**
(21) Application number: 99964508.8
(22) Date of filing: 23.11.1999
(51) Int. Cl.: A61L 9/20, C02F 1/32, H01J 65/04

(54) **MICROWAVE ACTUATED ULTRAVIOLET LIGHT SOURCE**
DURCH MIKROWELLEN BETÄTIGTE ULTRAVIOLETT-LICHTQUELLE
SOURCE D'ULTRAVIOLET ACTIONNE PAR MICROONDES

(30) Priority: 28.11.1998 GB 9826033; 29.07.1999 GB 9917661
(43) Date of publication of application: 04.10.2001
(73) Proprietor: Quay Technologies Ltd., Cookham Dean, Berks SL6 9PN (GB)
(72) Inventor: LUCAS, James, Blundellsands, Liverpool L23 7XA (GB); MORUZZI, James Lodovico, Cookhaven Dean, Berkshire SL6 9PN (GB)
(74) Representative: Pike, Christopher Gerard
(86) International application number: EP9909173
(87) International publication number: WO00032244

(56) References cited:
- WO-A-96/09842
- US-A- 3 911 318
- PATENT ABSTRACTS OF JAPAN vol. 010, no. 203 (C-360), 16 July 1986 (1986-07-16) & JP 61 046290 A (TOSHIBA CORP), 6 March 1986 (1986-03-06)

## Description

### Background to the Invention

It is known to use ultraviolet (UV) radiation in sterilisation systems for use in the purification of water and the sanitisation of items. The UV radiation and any ozone produced by the UV radiation with oxygen in the air acts to kill bacteria and germs. It is also known to employ microwave energy to excite the source of UV radiation in such systems.

One problem with known systems is that it is difficult to safely provide sufficient excitation energy to the UV source and difficult to effectively transfer that energy to the substance to be sterilised. It is therefore difficult to arrange systems for high energy, high throughput sterilisation purposes.

There is now described a steriliser which enables efficient, high throughput sterilisation to be conducted. The steriliser comprises a UV lamp which is excited by a microwave energy source. The lamp is enclosed by a waveguide comprising UV transparent material.

WO96/402 describes an electrodeless sterilisation apparatus comprising a UV lamp which is excited by a microwave energy source. The UV lamp is shaped to define a sterilisation passage therein. In use, the substance to be sterilised is passed through the sterilisation passage in the lamp. It may be appreciated that the size and geometry of the sterilisation passage will inevitably place limitations on the types of substances which may be sterilised using this apparatus and on the throughput achievable. It is also believed that direct contact of water with the lamp may affect the sterilisation capability of the lamp. Further, from a safety standpoint it is clearly undesirable that any breakage of the lamp may result in toxic vapour elements (e.g. mercury) contacting the substance to be sterilised.

US-A-5,166,528 describes a microwave excited ultraviolet steriliser for surface sterilisation of articles such as baby bottles and contact lenses. The steriliser comprises a plurality of UV bulbs which directly emit radiation to the articles.

US-A-5,141,636 describes a water purification system in which water is flowed along a flow path past a UV source. No mention is made of microwave excitation of the UV source.

WO97/3562 describes a steriliser employing a microwave-actuated UV energy source as the sterilisation means. No waveguide is provided between the UV energy source and the substance to be sterilised.

The WO-A-9 609 842 describes a sterilization apparatus comprising a microwave source and an ultraviolet light source surrounded by a hollow column.

### Summary of the Invention

The invention provides an ultraviolet light source comprising
an ultraviolet lamp;
a microwave energy source for exciting said ultraviolet lamp; and
a waveguide for guiding microwave energy originating from said microwave energy source to the ultraviolet lamp, wherein said waveguide is UV transparent and wholly surrounds the ultraviolet lamp,
various applications according to independant claims 23, 25 to 30 and 32 are also provided.

By UV transparent waveguide it is meant a waveguide that is substantially transparent to ultraviolet radiation, typically having a transparency of greater than 90%, preferably greater than 95% to UV radiation.

Suitably, the ultraviolet lamp has no electrode. That is to say it is an electrodeless lamp such as one comprising a partially evacuated tube comprising an element or mixtures of elements in vapour form. Mercury is a preferred element for this purpose, but alternatives include mixtures of inert gases with mercury compounds, sodium and sulphur. Preferably, the dominant wavelength produced by the lamp is 254nm.

In one aspect, the waveguide controls the flow of microwave energy. Control of the microwave energy which passes through the waveguide is useful in embodiments of the invention which make of both UV and microwave radiation in the sterilisation process.

In another aspect, the waveguide blocks the flow of microwave energy.

Suitably, the enclosure comprises quartz or a UV-transparent plastic material.

Suitably, the waveguide comprises a conducting material. The conducting material may be integral, or applied as a coating or liner. The liner may directly contact the inner surface of the enclosure or be spaced therefrom.

Suitably, the waveguide comprises a conducting mesh. Preferably, the conducting mesh comprises a material selected from the group consisting of copper, aluminium and stainless steel.

Suitably, the ultraviolet lamp has an elongate form such as a cigar-shape.

Suitably, the transparent waveguide has a cylindrical or rectangular form.

Suitably, the ultraviolet lamp has an operating temperature of less than 70°C.

Suitably, the microwave energy source comprises a magnetron. Alternative sources are envisaged such as solid state devices.

Suitably, the device additionally comprises a pathguide to guide the microwave energy from the microwave energy source to the ultraviolet lamp.

In one aspect the pathguide defines an essentially linear path for the microwave energy.

In another aspect, the pathguide defines a non-linear path such as a path defining at least one right angle.

Suitably, the device additionally comprises a housing for said enclosure. Preferably, the housing has an inlet and an outlet and the housing is shaped to guide fluid flow from the inlet, past the enclosure to the outlet. Preferably, the fluid comprises water or air. Suitably, the steriliser additionally comprises a pump for pumping fluid from the inlet, past the enclosure to the outlet. Alternatively, gravity may be utilised to encourage fluid flow.

Preferably, the ultraviolet lamp has no electrode.

According to a further aspect of the present invention there is provided a method of sterilising a substance comprising applying microwave energy to an ultraviolet lamp to produce ultraviolet radiation; and exposing the substance to said ultraviolet radiation according to claim 32.

In one aspect, the substance flows past the enclosure.

### Brief description of the drawings

Preferred embodiments of the steriliser comprising a light source in accord with the present invention will now be described with reference to the accompanying drawings in which:
Figure 1. is a schematic representation of a first steriliser herein suitable for water purification purposes;
Figures 2a and 2b are schematic representations of second and third sterilisers herein suitable for use in water purification;
Figures 3a and 3b are schematic representations of fourth and fifth sterilisers herein suitable for use in air purification;
Figure 4. is a schematic representation of a sixth steriliser herein suitable for use in combined UV and microwave sterilisation methods.

### Detailed description of the invention

The present invention is here described by means of examples, which constitute possible embodiments of the invention.

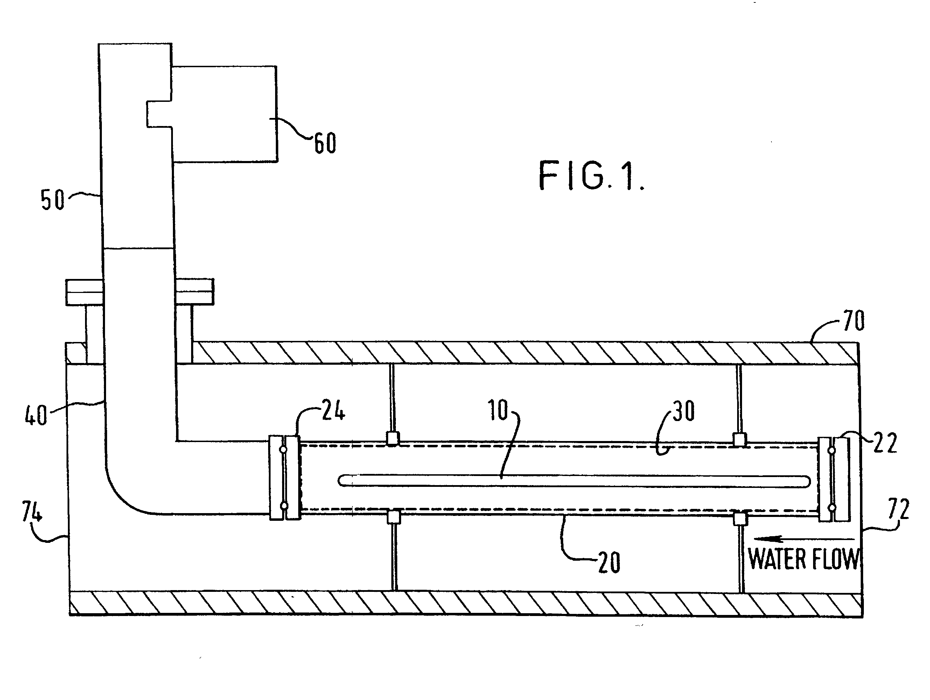

Figure 1. shows a steriliser comprising an ultraviolet lamp 10 enclosed by cylindrical enclosure 20. The cylindrical walls of the enclosure 20 form a waveguide and are comprised of quartz material which is transparent to UV radiation. A conducting copper mesh 30 is provided to the inner surface of the waveguide. First end of the cylindrical enclosure has blocking end flange 22 provided thereto. The second end is provided with coupling flange 24 which couples with right angled waveguide 40 which in turn connects with rectangular waveguide 50. Magnetron 60 acts as a microwave energy source to feed microwaves into the rectangular waveguide 50, thence into the right angled waveguide 40 and finally to the ultraviolet lamp 10 which is excited thereby.

The enclosure 20 is within tubular housing 70. The housing 70 has a water inlet 72 and a water outlet 74 provided thereto. In use, water flows from the inlet 72 past the enclosure 20 and towards the outlet 74. As the water flows past the enclosure 20 it is irradiated with UV radiation produced by the ultraviolet lamp 10. The radiation itself passes through the UV transparent walls of the enclosure 120a, 120b to contact the water.

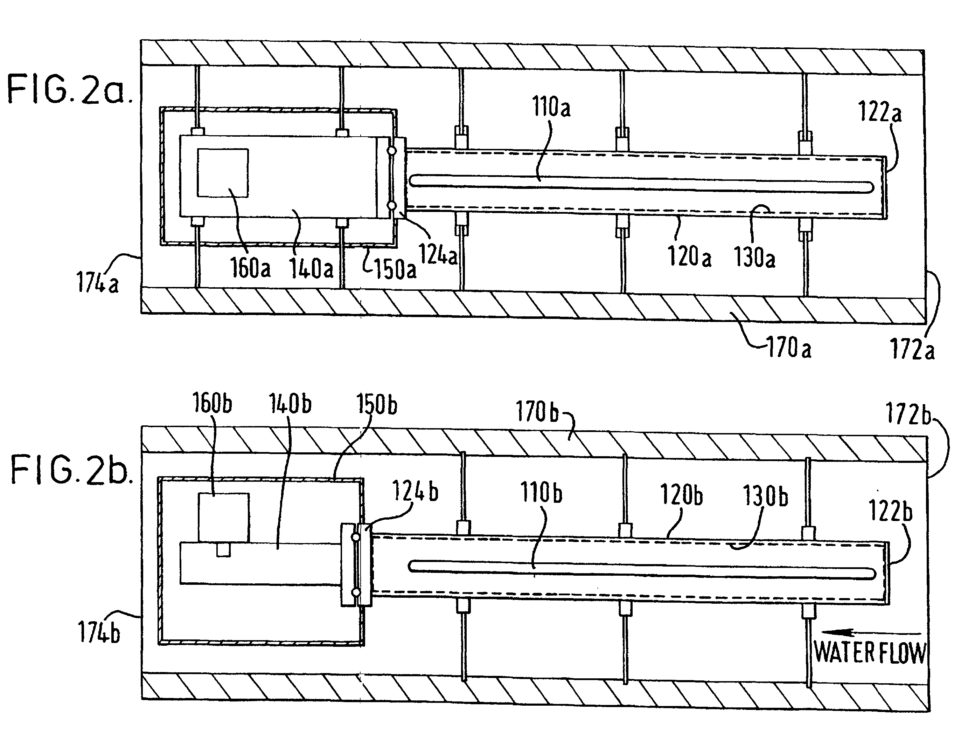

Figures 2a and 2b show related santisers herein. Both comprise ultraviolet mercury discharge lamp 110a, 110b enclosed by cylindrical enclosure 120a, 120b. The cylindrical walls of the enclosure 120a, 120b form a waveguide and are comprised of quartz material which is transparent to UV radiation. A conducting copper mesh 130a, 130b is provided to the inner surface of the waveguide. The enclosure 120a, 120b has air or nitrogen circulating therein. First end of the cylindrical enclosure has blocking end flange 122a, 122b provided thereto. The second end is provided with coupling flange 124a, 124b which couples with water-tight chamber 150a, 150b which contains brass waveguide 140a, 140b and magnetron 160a, 160b. The magnetron 160a, 160b acts as a microwave energy source to feed microwaves into the brass waveguide 140a, 140b and thence to the ultraviolet lamp 110a, 110b which is excited thereby.

The enclosure 120a, 120b is within tubular housing 170a, 170b. The housing 170a, 170b has a water inlet 172a, 172b and a water outlet 174a, 174b provided thereto. In use, water flows from the inlet 172a, 172b past the enclosure 120a, 120b and towards the outlet 174a, 174b. As the water flows past the enclosure 120a, 120b it is irradiated with UV radiation produced by the ultraviolet lamp 110a, 110b. The radiation itself passes through the UV transparent walls of the enclosure 120a, 120b to contact the water.

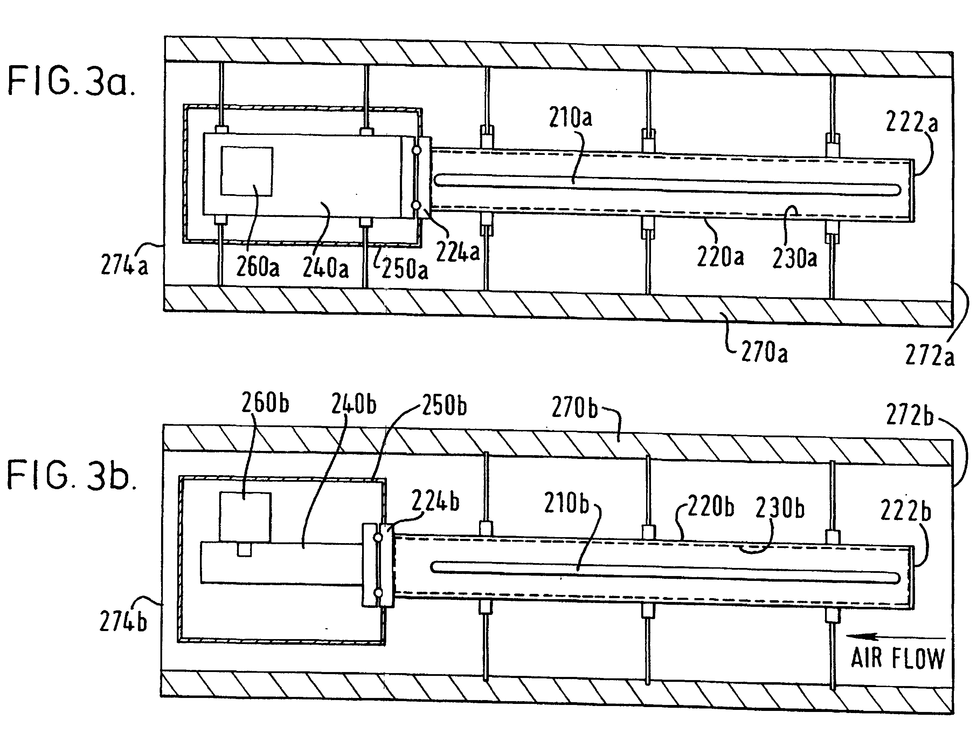

Figures 3a and 3b show sanitisers similar in structure to the sanitisers of Figures 2a and 2b but for use in air purification. Both comprise ultraviolet mercury discharge lamp 210a, 210b enclosed by cylindrical enclosure 220a, 220b. The cylindrical walls of the enclosure 220a, 220b form a waveguide and are comprised of quartz material which is transparent to UV radiation. A conducting copper mesh 230a, 230b is provided to the inner surface of the waveguide. The enclosure 220a, 220b has air or nitrogen circulating therein. First end of the cylindrical enclosure has blocking end flange 222a, 222b provided thereto. The second end is provided with coupling flange 224a, 224b which couples with airtight chamber 250a, 250b containing brass waveguide 240a, 240b and magnetron 260a, 260b. The magnetron 260a, 260b acts as a microwave energy source to feed microwaves into brass waveguide 240a, 240b and thence to the ultraviolet lamp 210a, 210b which is excited thereby.

The enclosure 220a, 220b is within tubular housing 270a, 270b. The housing 270a, 270b has an air inlet 272a, 272b and an air outlet 274a, 274b provided thereto. In use, air flows from the inlet 272a, 272b past the enclosure 220a, 220b and towards the outlet 274a, 274b. As the air flows past the enclosure 220a, 220b it is irradiated with UV radiation produced by the ultraviolet lamp 210a, 210b. The radiation itself passes through the UV transparent walls of the enclosure 220a, 220b to contact the air killing the bacteria and germs therein.

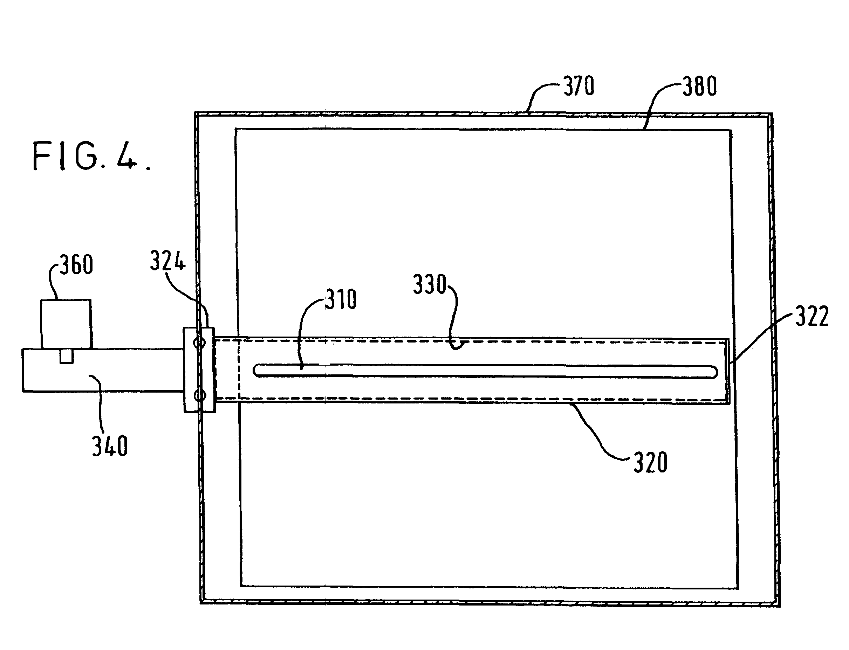

Figure 4 shows a cabinet steriliser herein suitable for use in sterilising objects such as medical instruments. Ultraviolet mercury discharge lamp 310 is enclosed by cylindrical enclosure 320. The cylindrical walls of the enclosure 320 form a waveguide and are comprised of quartz material which is transparent to UV radiation but only partially transparent to microwave radiation. A conducting copper mesh 330 is provided to the inner surface of the waveguide. The enclosure 320 optionally has air or nitrogen circulating therein. First end of the cylindrical enclosure has blocking end flange 322 provided thereto. The second end is provided with coupling flange 324 which couples with linear pathguide 340 which in tum connects with magnetron 360. The magnetron 360 acts as a microwave energy source to feed microwaves into pathguide 340 and thence to the ultraviolet lamp 310 which is excited thereby.

The enclosure 320 is within housing 370 which has an entry door 380 provided thereto. In use, items to be sterilised, which can include metal items, are placed in the housing 370. The items are irradiated with UV radiation produced by the ultraviolet lamp 310 and by microwave radiation deriving from the magnetron 360. The radiation itself, passes through the UV transparent and microwave partially transparent walls of the enclosure 320 to contact the items. Optionally, the housing 370 may be provided with UV transparent shelves for the items. An inner reflective lining, for example an aluminium foil lining, may also be provided to the housing 370.

The device of the present invention is suitable for use in sterilising water for human consumption; sterilising waste water and sewage; sterilising metallic and non-metallic objects including medical instruments; sterilising air in buildings such as hospitals, offices and homes; curing glues and special inks; erasing eproms; and prolonging the shelf-life of foodstuffs by killing bacteria on the surface of the goods.

The device of the present invention is suitable in one aspect for use in air-conditioning systems for use in vehicles such as cars, lorries and buses. The sanitiser will be sized and shaped to fit within the air-conditioning system of the vehicle and will typically therefore have a size less than the size it would possess when used in large scale air and water treatment applications.

The ultraviolet light produced by the sanitiser herein may additionally be channelled as a light source of high intensity. Suitable uses would include lighting within buildings and lighting for vehicles such as cars, lorries and buses.

## Claims

1. An ultraviolet light source comprising
an ultraviolet lamp;
a microwave energy source for exciting said ultraviolet lamp; and
a waveguide for guiding microwave energy originating from said microwave energy source to the ultraviolet lamp, wherein said waveguide is UV transparent and wholly surrounds the ultraviolet lamp.

2. An ultraviolet light source according to claim 1, wherein the ultraviolet lamp has no electrode.

3. An ultraviolet light source according to claim 2, comprising an element or mixture of elements in vapour form.

4. An ultraviolet light source according to claim 3, wherein said element or mixture of elements comprises mercury, sodium, sulphur or mixtures of inert gases with mercury compounds.

5. An ultraviolet light source according to any of claims 2 to 4 having a dominant wavelength of 254nm.

6. An ultraviolet light source according to any of claims 1 to 5, wherein the waveguide controls the flow of microwave energy.

7. An ultraviolet light source according to either of claims 1 to 5, wherein the waveguide blocks the flow of microwave energy.

8. An ultravlolet light source according to any of claims 1 to 7, wherein the wave guide comprises quartz or a UV-transparent plastic material.

9. An ultraviolet light source according to any of claims 1 to 8, wherein the waveguide comprises a conducting material.

10. An ultraviolet light source according to claim 9, wherein the waveguide comprises a conducting mesh.

11. An ultraviolet light source according to claim 10, wherein the conducting mesh comprises a material selected from the group consisting of copper, aluminium and stainless steel.

12. An ultraviolet light source according to any of claims 1 to 11, wherein the ultraviolet lamp has an elongate form.

13. An ultraviolet light source according to any of claims 1 to 12, wherein the transparent waveguide has a cylindrical or rectangular form.

14. An ultraviolet light source according to any of claims 1 to 13, wherein the ultraviolet lamp has an operating temperature of less than 70°C.

15. An uftraviofet light source according to any of claims 1 to 14, wherein the microwave energy source comprises a magnetron.

16. An ultraviolet light source according to any of claims 1 to 15, additionally comprising a pathguide to guide the microwave energy from the microwave energy source to the ultraviolet lamp.

17. An ultraviolet light source according to claim 16, wherein the pathguide defines an essentially linear path.

18. An ultraviolet light source according to claim 16, wherein the pathguide defines a non-linear path.

19. An ultraviolet light source according to any of claims 1 to 18 additionally comprising a housing for said wave guide.

20. An ultraviolet light source according to claim 19, wherein the housing has an inlet and an outlet and the housing is shaped to guide fluid flow from the inlet, past the enclosure to the outlet.

21. An ultraviolet light source according to claim 20, wherein said fluid comprises water or air.

22. An ultraviolet light source according to either of claims 20 or 21, additionally comprising a pump for pumping fluid from the inlet, past the enclosure to the outlet.

23. Use of an ultraviolet light source according to any of claims 1 to 22 for sterilising a substance.

24. Use according to claim 23, wherein said substance is selected from the group consisting of water for human consumption; waste water; sewage; metallic and non-metallic objects; and air.

25. Use of an ultraviolet light source according to any of claims 1 to 22 for curing glues and inks.

26. Use of an ultraviolet light source according to any of claims 1 to 22 for erasing eproms.

27. Use of an ultraviolet light source according to any of claims 1 to 22 for killing bacteria on the surface of goods.

28. Air conditioning system comprising an ultraviolet light source according to any of claims 1 to 22.

29. High intensity lighting system comprising an ultraviolet light source according to any of claims 1 to 22.

30. A lamp arrangement comprising
an ultraviolet lamp, said lamp being excitable by microwave energy; and
a waveguide for guiding microwave energy originating from a microwave energy source to the ultraviolet lamp,
wherein said waveguide is UV transparent and wholly surrounds the ultraviolet lamp.

31. A lamp arrangement according to claim 30, wherein the ultraviolet lamp has no electrode.

32. A method of sterilising a substance comprising
guiding microwave energy from a microwave energy source to an ultraviolet lamp to produce ultraviolet radiation; and
exposing the substance to said ultraviolet radiation, wherein
a waveguide guides said microwave energy to said ultraviolet lamp and said waveguide is UV transparent and wholly surrounds the ultraviolet lamp.

33. A method according to claim 32, wherein the substance flows past said enclosure.

## Revendications

**1.** Source de lumière ultraviolette comprenant :
une lampe à ultraviolets ;
une source d'énergie microonde pour exciter ladite lampe à ultraviolets ; et
un guide d'ondes pour guider l'énergie microonde provenant de ladite source d'énergie microonde vers la lampe à ultraviolets, dans laquelle ledit guide d'ondes est transparent aux UV et entoure totalement la lampe à ultraviolets.

**2.** Source de lumière ultraviolette suivant la revendication 1, dans laquelle la lampe à ultraviolets n'a pas d'électrode.

**3.** Source de lumière ultraviolette suivant la revendication 2, comprenant un élément ou un mélange d'éléments sous forme de vapeur.

**4.** Source de lumière ultraviolette suivant la revendication 3, dans laquelle ledit élément ou ledit mélange d'éléments comprend du mercure, du sodium, du soufre ou des mélanges de gaz inertes avec des composés du mercure.

**5.** Source de lumière ultraviolette suivant l'une quelconque des revendications 2 à 4 ayant une longueur d'onde dominante de 254 nm.

**6.** Source de lumière ultraviolette suivant l'une quelconque des revendications 1 à 5, dans laquelle le guide d'ondes contrôle le flux d'énergie microonde.

**7.** Source de lumière ultraviolette suivant l'une quelconque des revendications 1 à 5, dans laquelle le guide d'ondes bloque le flux d'énergie microonde.

**8.** Source de lumière ultraviolette suivant l'une quelconque des revendications 1 à 7, dans laquelle le guide d'ondes comprend du quartz ou un matériau plastique transparent aux UV.

**10.** Source de lumière ultraviolette suivant la revendication 9, dans laquelle le guide d'ondes comprend un treillis conducteur.

**11.** Source de lumière ultraviolette suivant la revendication 10, dans laquelle le treillis conducteur comprend un matériau choisi dans le groupe consistant en cuivre, aluminium et acier inoxydable.

**12.** Source de lumière ultraviolette suivant l'une quelconque des revendications 1 à 11, dans laquelle la lampe à ultraviolets a une forme allongée.

**13.** Source de lumière ultraviolette suivant l'une quelconque des revendications 1 à 12, dans laquelle le guide d'ondes transparent a une forme cylindrique ou rectangulaire.

**14.** Source de lumière ultraviolette suivant l'une quelconque des revendications 1 à 13, dans laquelle la lampe à ultraviolets a une température de fonctionnement inférieure à 70°C.

**15.** Source de lumière ultraviolette suivant l'une quelconque des revendications 1 à 14, dans laquelle la source d'énergie microonde comprend un magnétron.

**16.** Source de lumière ultraviolette suivant l'une quelconque des revendications 1 à 15, comprenant de plus un guide d'ondes pour guider l'énergie microonde depuis la source d'énergie microonde jusqu'à la lampe à ultraviolets.

**17.** Source de lumière ultraviolette suivant la revendication 16, dans laquelle le guide d'ondes définit un chemin essentiellement linéaire.

**18.** Source de lumière ultraviolette suivant la revendication 16, dans laquelle le guide d'ondes définit un chemin non linéaire.

**19.** Source de lumière ultraviolette suivant l'une quelconque des revendications 1 à 18 comprenant de plus un carter pour ledit guide d'onde.

**20.** Source de lumière ultraviolette suivant la revendication 19, dans laquelle le carter a une entrée et une sortie et le carter a une forme destinée à guider le flux de fluide depuis l'entrée, le long de l'enceinte et jusqu'à la sortie.

**21.** Source de lumière ultraviolette suivant la revendication 20, dans laquelle ledit fluide comprend l'eau ou l'air.

**22.** Source de lumière ultraviolette suivant l'une ou l'autre des revendications 20 ou 21, comprenant de plus une pompe pour pomper du fluide depuis l'entrée, le long de l'enceinte jusqu'à la sortie.

**23.** Utilisation d'une source de lumière ultraviolette suivant l'une quelconque des revendications 1 à 22, pour la stérilisation d'une substance.

**24.** Utilisation suivant la revendication 23, dans laquelle ladite substance est choisie dans le groupe consistant en eau pour la consommation humaine ; eaux résiduaires ; eaux d'égout ; objets métalliques et non métalliques ; et l'air.

**25.** Utilisation d'une source de lumière ultraviolette suivant l'une quelconque des revendications 1 à 22, pour le durcissement de colles et d'encres.

**26.** Utilisation d'une source de lumière ultraviolette suivant l'une quelconque des revendications 1 à 22, pour l'effacement de mémoires programmables de type eprom.

**27.** Utilisation d'une source de lumière ultraviolette suivant l'une quelconque des revendications 1 à 22, pour la destruction de bactéries présentes sur la surface de marchandises.

**28.** Système de conditionnement d'air comprenant une source de lumière ultraviolette suivant l'une quelconque des revendications 1 à 22.

**29.** Système d'éclairage de haute intensité comprenant une source de lumière ultraviolette suivant l'une quelconque des revendications 1 à 22.

**30.** Dispositif de lampe comprenant :
une lampe à ultraviolets, ladite lampe pouvant être excitée par une énergie microonde ; et
un guide d'ondes pour guider l'énergie microonde provenant d'une source d'énergie microonde jusqu'à la lampe à ultraviolets,
dans lequel ledit guide d'ondes est transparent aux UV et entoure totalement la lampe à ultraviolets.

**31.** Dispositif de lampe suivant la revendication 30, dans lequel la lampe à ultraviolets n'a pas d'électrode.

**32.** Procédé pour la stérilisation d'une substance comprenant :
le guidage d'une énergie microonde à partir d'une source d'énergie microonde jusqu'à une lampe à ultraviolets pour produire un rayonnement ultraviolet ; et
l'exposition de la substance audit rayonnement ultraviolet,
dans lequel un guide d'ondes guide ladite énergie microonde jusqu'à ladite lampe à ultraviolets et ledit guide d'ondes est transparent aux UV et entoure totalement la lampe à ultraviolets.

**33.** Procédé suivant la revendication 32, dans lequel la substance s'écoule le long de ladite enceinte.

## Patentansprüche

1. Ultraviolettlichtquelle, umfassend:
eine Ultraviolettlampe;
eine Mikrowellenenergiequelle zum Anregen der Ultraviolettlampe; und
eine Wellenführung zum Führen der Mikrowellenenergie, die von der Mikrowellenenergiequelle ausgeht, zu der Ultraviolettlampe, wobei die Wellenführung UV transparent ist und die Ultraviolettlampe vollständig umgibt.

2. Ultraviolettlichtquelle nach Anspruch 1, worin die Ultraviolettlampe keine Elektrode besitzt.

3. Ultraviolettlichtquelle nach Anspruch 2, umfassend ein Element oder eine Mischung von Elementen in Dampfform.

4. Ultraviolettlichtquelle nach Anspruch 3, worin das Element oder die Mischung von Elementen Quecksilber, Natrium, Schwefel oder Mischungen inerter Gase mit Quecksilberverbindungen aufweist.

5. Ultraviolettlichtquelle nach einem der Ansprüche 2 bis 4, die eine maßgebende Wellenlänge von 254 nm besitzt.

6. Ultraviolettlichtquelle nach einem der Ansprüche 1 bis 5, worin die Wellenführung den Strom der Mikrowellenenergie regelt .

7. Ultraviolettlichtquelle nach einem der Ansprüche 1 bis 5, worin die Wellenführung in Strom von Mikrowellenenergie blockt.

8. Ultraviolettlichtquelle nach einem der Ansprüche 1 bis 7, worin die Wellenführung Quarz oder UV transparentes Kunststoffmaterial aufweist.

9. Ultraviolettlichtquelle nach einem der Ansprüche 1 bis 8, worin die Wellenführung ein leitendes Material aufweist.

10. Ultraviolettlichtquelle nach Anspruch 9, worin die Wellenführung ein leitendes Netz aufweist.

11. Ultraviolettlichtquelle nach Anspruch 10, worin das leitende Netz ein Material aufweist, das aus der Gruppe ausgewählt ist, die aus Kupfer, Aluminium und rostfreiem Stahl besteht.

12. Ultraviolettlichtquelle nach einem der Ansprüche 1 bis 11, worin die Ultraviolettlampe eine längliche Form besitzt.

13. Ultraviolettlichtquelle nach einem der Ansprüche 1 bis 12, worin die transparente Wellenführung eine zylindrische oder rechteckige Form besitzt.

14. Ultraviolettlichtquelle nach einem der Ansprüche 1 bis 13, worin die Ultraviolettlampe eine Betriebstemperatur von weniger als 70° C besitzt.

15. Ultraviolettlichtquelle nach einem der Ansprüche 1 bis 14, worin die Mikrowellenenergiequelle ein Magnetron aufweist.

16. Ultraviolettlichtquelle nach einem der Ansprüche 1 bis 15, die zusätzlich eine Pfadführung aufweist, um die Mikrowellenenergie von der Mikrowellenenergiequelle zu der Ultraviolettlampe zu führen.

17. Ultraviolettlichtquelle nach Anspruch 16, worin die Pfadführung einen im wesentlichen linearen Pfad definiert.

18. Ultraviolettlichtquelle nach Anspruch 16, worin die Pfadführung einen nicht linearen Pfad definiert.

19. Ultraviolettlichtquelle nach einem der Ansprüche 1 bis 18, die zusätzlich ein Gehäuse für die Wellenführung aufweist.

20. Ultraviolettlichtquelle nach Anspruch 19, worin das Gehäuse einen Einlass und einen Auslass besitzt, und das Gehäuse ist derart geformt, um den Fluidstrom von dem Einlass hinter die Umschließung zu dem Auslass zu führen.

21. Ultraviolettlichtquelle nach Anspruch 20, worin das Fluid Wasser oder Luft aufweist.

22. Ultraviolettlichtquelle nach einem der Ansprüche 20 oder 21, die zusätzlich eine Pumpe zum Pumpen von Fluid von dem Einlass hinter die Umschließung zu dem Auslass aufweist.

23. Verwendung einer Ultraviolettlichtquelle nach einem der Ansprüche 1 bis 22 zum Sterilisieren einer Substanz.

24. Verwendung nach Anspruch 22, worin die Substanz aus der Gruppe ausgewählt wird, die aus Wasser für den menschlichen Verbrauch; Schmutzwasser; Abwasser; metallischen und nicht metallischen Gegenstände; und Luft besteht.

25. Verwendung einer Ultraviolettlichtquelle nach einem der Ansprüche 1 bis 22 zum Aushärten bzw. Trocknen von Klebstoffen und Tinten.

26. Verwendung einer Ultraviolettlichtquelle nach einem der Ansprüche 1 bis 22 zum Löschen von Eproms.

27. Verwendung einer Ultraviolettlichtquelle nach einem der Ansprüche 1 bis 22 zum Töten von Bakterien auf der Oberfläche von Waren.

28. Luftklimatisierungssystem, umfassend eine Ultraviolettlichtquelle nach einem der Ansprüche 1 bis 22.

29. Hochintensitäts-Beleuchtungssystem, umfassend eine Ultraviolettlichtquelle nach einem der Ansprüche 1 bis 22.

30. Lampenanordnung, umfassend:
eine Ultraviolettlampe, wobei die Lampe durch Mikrowellenenergie anregbar ist; und
eine Wellenführung zum Führen von Mikrowellenenergie, die von einer Mikrowellenenergiequelle ausgeht, zu der Ultraviolettlampe,
worin die Wellenführung UV transparent ist und die Ultraviolettlampe vollständig umgibt.

31. Lampenanordnung nach Anspruch 30, worin die Ultraviolettlampe keine Elektrode besitzt.

32. Verfahren zum Sterilisieren einer Substanz, umfassend:
Führen von Mikrowellenenergie von einer Mikrowellenenergiequelle zu einer Ultraviolettlampe, um Ultraviolettstrahlung zu erzeugen; und
Aussetzen der Substanz der Ultraviolettstrahlung, worin
eine Wellenführung die Mikrowellenenergie zu der Ultraviolettlampe führt, und die Wellenführung ist UV transparent und umgibt vollständig die Ultraviolettlampe.

33. Verfahren nach Anspruch 32, worin die Substanz hinter die Umschließung strömt.
